# EUROPEAN PATENT APPLICATION

(11) **EP 4 112 044 A1**
(43) Date of publication of application: **04.01.2023**
(21) Application number: 21181438.9
(22) Date of filing: 24.06.2021
(51) Int. Cl.: A61K 9/14, A61K 31/436, A61K 31/506, A61K 31/522, A61K 31/573, A61K 31/58, A61K 31/7088, A61K 38/13, A61K 8/02, A61K 8/35, A61K 8/49, A61P 17/14, A61Q 7/00, A61Q 19/00, A61K 9/00

(54) **COMPOSITION FOR PARTICLE-MEDIATED TRANSPORT OF A DISSOLVED ACTIVE AGENT INTO HAIR FOLLICLES**

(71) Applicant: Charité - Universitätsmedizin Berlin, 10117 Berlin (DE); Philipps Universität Marburg, 35032 Marburg (DE)
(72) Inventor: LADEMANN, Jürgen, 15517 Fürstenwalde (DE); MEINKE, Martina, 14193 Berlin (DE); KLEIN, Anna-Lena, 12439 Berlin (DE); BUSCH, Loris, 14057 Berlin (DE); KECK, Cornelia, 14548 Berlin (DE); PELIKH, Olga, 35057 Marburg (DE)
(74) Representative: Hertin und Partner Rechts- und Patentanwälte PartG mbB

(57) **Abstract**

The invention relates to a composition for the transport of a dissolved active agent into hair follicles, comprising an active agent dissolved in a topical dispersion medium, and submicron particles. In one aspect, the invention relates to a pharmaceutical composition for use in transporting a dissolved pharmacologically active therapeutical and/or prophylactic agent into hair follicles comprising a pharmacologically active therapeutic and/or prophylactic agent dissolved in a topical dispersion medium, and submicron particles. In embodiments, the active agent is not chemically coupled to the submicron particles. In a further aspect, the composition is a cosmetic composition comprising a cosmetically active agent, and the cosmetic composition can be used in a cosmetic method, wherein the cosmetic composition is applied to an area of skin with hair follicles.

## Description

The invention relates to a composition for the transport of a dissolved active agent into hair follicles, comprising an active agent dissolved in a topical dispersion medium, and submicron particles. In one aspect, the invention relates to a pharmaceutical composition for use in transporting a dissolved pharmacologically active therapeutical and/or prophylactic agent into hair follicles comprising a pharmacologically active therapeutic and/or prophylactic agent dissolved in a topical dispersion medium, and submicron particles. In embodiments, the active agent is not chemically coupled to the submicron particles. In a further aspect, the composition is a cosmetic composition comprising a cosmetically active agent, and the cosmetic composition can be used in a cosmetic method, wherein the cosmetic composition is applied to an area of skin with hair follicles.

### BACKGROUND OF THE INVENTION

The skin is a very stable barrier, so that it is very difficult to transport topically applied substances through the skin barrier. Hair follicles have been identified as interesting target sites for delivering topically applied substances such as pharmaceutically active substances, for example for topical vaccinations or agents used in the field of regenerative medicine, but also for delivery of cosmetic agents. In recent years, it could be shown that particles penetrate very effectively into the hair follicles.

It was found that particles penetrate hair follicles after dermal application, while this is not the case for particle-free formulations ^{1,2}. Furthermore, it has been shown that this happens especially when the particles have a size < 1 µm and particularly when the particle sizes are in the range between 500 - 800 nm ³. The underlying mechanism for the transport of particles into hair follicles is the "ratchet effect" ⁴.

The hair follicle has been shown to be a relevant penetration pathway for particles as well as an important long-term reservoir for substances that are comprised by or bound to the particle. It has been demonstrated that the penetration depth of the particles can be influenced by their size resulting in the possibility of a differentiated targeting of specific follicular structures. Accordingly, topically applied nanoparticles or nanocrystals with a diameter of approx. 600 nm can thus be effectively captured and transported deep into the hair follicle.

However, non-particulate substances including dissolved active agents, for example for pharmaceutical or cosmetic purposes, do practically not penetrate the hair follicle and remain on the surface of the skin upon topical application. Accordingly, upon topical application penetration of the skin by dissolved agents is very limited.

For many agents it is not possible or disadvantageous to couple or comprise them to submicron particles that can penetrate the hair follicle, and therefore delivery through direct coupling or inclusion into particles is not an option. Still, delivery of such dissolved substances into the hair follicle upon topical application would be highly advantageous since substances that penetrate deep into the hair follicle can reach the tight junctions in the lower part of the follicle and can penetrate the surrounding tissue. Furthermore, the follicle could serve as a reservoir for dissolved agents, which can be highly advantageous for reducing the frequency of topical application of the agent, while still being highly effective.

As is evident from the prior art, although it is possible to efficiently deliver particles to the hair follicle, there are to date no efficient means for delivering dissolved active agents, such as dissolved drug molecules or cosmetically active molecules, to hair follicles, for example for effective and prolonged penetration of the skin.

### SUMMARY OF THE INVENTION

In light of the prior art the technical problem underlying the present invention is to provide alternative and/or improved means for delivering dissolved active agents into hair follicles.

This problem is solved by the features of the independent claims. Preferred embodiments of the present invention are provided by the dependent claims.

The invention therefore relates to a composition for the transport of a dissolved active agent into hair follicles, comprising
a. an active agent dissolved in a topical dispersion medium, and
b. submicron particles.

The present invention is based on the entirely surprising finding that dissolved active agents, such as small chemical compounds or large biologic molecules, such as a biologic polymer, such as a (recombinant) protein, can be delivered to a hair follicle when it is administered to the skin in a composition comprising submicron particles that are dispersed in the dispersion medium. It was entirely unexpected that the presence of such particles in the topical dispersion medium can enhance penetration of the dissolved active agent, which is not coupled to the particles, for example by a chemical bond or any specific linkage, into the follicle.

So far, it was believed that the physical properties of solid particles of submicron size (particles which are 1 micrometer (µm) or smaller) in conjunction with the anatomical properties of hair follicles enables specific penetration of the particle into the follicle, while the medium containing the particles will remain on the surface of the skin. Accordingly, the particles have been viewed as direct delivery vehicles for active agents into the follicle, wherein the particles comprise or are physically attached to the respective active agent. This means, that it was generally accepted that a direct coupling between an active agent and a particle is required to enable delivery of the agent to the hair follicle.

However, in the context of the present invention it was unexpectedly discovered that submicron particles also enable the delivery of active agents that are not directly coupled to the particles and that are not incorporated in the particles when the agent is dissolved in the dispersion medium containing the particles. This kind of facilitation of follicle penetration of dissolved active agents that are not attached to the particles can be referred to as indirect facilitation of penetration. In this context, the particles may be regarded as indirect delivery/penetration mediators.

The observed effect is particularly surprising since the hair follicle comprises sebaceous glands that produce sebum, which is an oily or waxy matter which lubricates the hair and skin. Therefore, the entry of liquids from the outside into the follicle is aggravated by the outside flowing sebum. It has been described how particles can still enter the follicle, in particular when the skin is massaged during or after application of the particle containing topical dispersion medium. However, it could not be expected that the dispersion medium and substances comprised therein are dragged along with the particles despite the sebum being present in the follicle shaft, flowing into the opposite direction. This is particularly true for hydrophilic media, which can be comprised by the dispersion medium in embodiments of the invention.

Surprisingly, it could be shown that the facilitation of agent penetration into the follicle is mostly independent of the properties of the active agent and/or the dispersion medium and rather depends on the presence of the submicron particles in the dispersion medium. In embodiments, the active agent is lipophilic, hydrophilic or amphiphilic. In embodiments, the composition comprises a dispersion medium that is suitable for dissolving a lipophilic, hydrophilic or amphiphilic active agent, such as a lipophilic, hydrophilic or amphiphilic medium. In embodiments, the agent is soluble and dissolved in the dispersion medium containing the submicron particles.

In embodiments, the dispersion medium comprises a solvent or solvent composition that is suitable for dissolving the active agent and that is compatible with the respective submicron particles.

In embodiments, the dissolved active agent is not associated with or specifically bound by the submicron particle. In particular, there is no chemical bond between the dissolved active agent and the particle.

In embodiments, the particle may comprise, be constituted of or be bound to a second active agent. In such embodiments, the composition may be used for delivering at least two active agents into hair follicles, one dissolved in the dispersion medium, and one couple or comprised by the particles of the composition.

In embodiments, the dispersion medium can comprise more than one dissolved active agents. Accordingly, in embodiments the composition of the invention can comprise multiple active agents, wherein one or more active agent are dissolved in the dispersion medium without being directly bound or coupled to the submicron particles, and wherein optionally the submicron particles also comprise or consist of an active agent and/or are directly (for example chemically) coupled to an active agent.

The present invention is highly useful for administering active agents to a subject via topical administration of an inventive composition to the skin. The hair follicle is a site where an agent can have a direct influence on the anatomical structures and cells of the follicle, for example in cosmetic and pharmaceutical applications concerning hair growth or hair loss, hair coloring and influencing the structure of the hair. Furthermore, the follicle can serve as an entry site for agents that are active in the skin, for example in lower layers of the skin such as the dermis or the hypodermis/subcutis, or that act systemically. In this context, the hair follicle can serve as a reservoir where an active agent can remain for prolonged time, while small amounts of the agent continuously diffuse into the surrounding tissue.

Therefore, in embodiments, delivery of dissolved active agents to hair follicles is highly advantageous to reduce the frequency of administration of the agent, or to prolong the activity of the active agent in comparison to compositions that fail to efficiently deliver the agent to the hair follicle and in which the agent mostly remains on the skin surface. In the context of the present invention, the active agent can remain in the follicle and therefore in/on the skin of a subject for a prolonged time in comparison to compositions that fail to deliver dissolved agents to the follicle.

In a further aspect, the present invention relates to a pharmaceutical composition for use in transporting a dissolved pharmacologically active therapeutical and/or prophylactic agent into hair follicles comprising
c. a pharmacologically active therapeutic and/or prophylactic agent dissolved in a topical dispersion medium, and
d. submicron particles.

The present invention is particularly suited for delivering pharmaceutically active agents to hair follicles of the skin. In embodiments, the pharmaceutically active agent acts directly in the hair follicle to influence the physiological functions of the anatomical structures of the follicle in the context of conditions related to hair or skin. In embodiments, the active agent is delivered to the hair follicle to enable more efficient activity of the agent on the surrounding tissue or to enhance penetration into the surrounding tissue. For example, the activity of topical anesthetics may be prolonged when the anesthetic is delivered as a dissolved active agent in the context of a composition of the present invention, since the active agent can remain in the follicle for a prolonged time.

In embodiments, the invention may reduce the amount of the active agent required for a specific application in comparison to conventional compositions for topical administration, since the agent is efficiently delivered to the hair follicle where it can remain active for longer periods of time, whereas in case of administration with conventional compositions the active agent remains of the skin surface where it is rapidly washed off.

In embodiments, the invention relates to a composition, wherein the active agent is not chemically coupled to the submicron particles.

In embodiments, the topical dispersion medium is a liquid, an oil, a cream, a lotion, a gel or a mixture of one or more of these, preferably a liquid such as water, ethanol or a surfactant solution. In principle, the present invention can use any kind of suitable and known topical dispersion medium and can comprise any kind of (organic) solvent and mixtures of solvents suitable for the respective application and active agent.

In principle, in embodiments of the invention, the combination particle type (P), active agent (A), and topical dispersion medium (DM) can be used for any combination of the following:
Submicron particles of the invention comprise, without limitation, active ingredient nanocrystals (possible active ingredients of which the nanocrystals consist are e.g. minoxidil, ciclosporin, finasteride, dudasteride, tacrolimus, caffeine, as well as active ingredients from the substance classes corticoids, flavonoids, antioxidants), lipid nanoparticles, liposomes, polymer nanoparticles - in each case loaded with active ingredients or unloaded, as well as particles made of inorganic materials (e.g. e.g. ZnO, silicates).

Active agents that can be comprised by the composition of the invention comprise, without limitation, substances from the substance group antioxidants (pure substances, extracts, mixtures, agents regulating hair growth, disinfectants, immunotherapeutics, etc.).

Dispersion medium that can be comprised by compositions of the invention comprise, without limitation:
- water and water with additives (solubilizers, surfactants, preservatives, stabilizers, humectants, colorants, etc.),
- alcohols (ethanol, propanol) in various dilutions with water and with possible additives,
- polyethylene glycols in different dilutions with water and/or alcohols with possible additives,
- lipids in different dilutions with water and/or alcohols with possible additives.

Specific preferred compositions of the invention can comprise the following combinations of P, A and DM:
- Tacrolimus nanocrystals (< 1µm) in water/ethanol mixture with addition of glycerol and surfactants with dissolved active ingredient - Caffeine.
- Tacrolimus nanocrystals (< 1µm) in water/ethanol mixture with addition of glycerol and surfactants with dissolved active ingredient - caffeine, green tea extract and further antioxidants
- Cyclosporin nanocrystals (< 1µm) in water/ethanol mixture with addition of glycerol and surfactants with dissolved active ingredient - caffeine, green tea extract and further antioxidants
- Finasteride nanocrystals (< 1µm) in water/ethanol mixture with addition of glycerol and surfactants with dissolved active ingredient - caffeine, green tea extract and other antioxidants
- Minoxidil nanocrystals (< 1µm) in water/ethanol mixture with addition of glycerol and surfactants with dissolved active ingredient - caffeine, green tea extract and further antioxidants
- Mixture of different nanocrystals (see above) in water/ethanol mixture with addition of glycerol and surfactants with dissolved active ingredient - caffeine, green tea extract and further antioxidants
- Lipid nanoparticles with water/ethanol mixture with addition of glycerol and surfactants with dissolved DNA, RNA, mRNA active ingredient
- Presdnisolone nanocrystals with addition of glycerol and surfactants with dissolved disinfectant
- Nanocrystals of antibiotic WS with addition of glycerol and surfactants with dissolved disinfectant or antibiotic.

In embodiments, the submicron particles of the composition of the invention have a diameter of 100-1000 nm, preferably 500-800 nm, most preferably about 600 nm. In the context of the invention, it is understood that a submicron particle is a particle of 1 µm maximal width or thickness. In case of an about spherical particle, this may be the diameter of the particle. It is understood that in the context of a (in particular non-spherical) particle of the invention, the term "diameter" refers to the maximal length/expansion of the particle from one side to the opposite side.

It was found that the invention functions with particles not larger than 1 µm. Preferably, the diameter of the submicron particles is 100 - 1000 nm. In embodiments, the diameter of the particles is about 20, 30, 40, 50, 60, 70, 80, 90, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, 500, 510, 520, 530, 540, 550, 560, 570, 580, 590, 600, 610, 620, 630, 640, 650, 660, 670, 680, 690, 700, 710, 720, 730, 740, 750, 760, 770, 780, 790, 800, 810, 820, 830, 840, 850, 860, 870, 880, 890, 900, 910, 920, 930, 940, 950, 960, 970, 980, 990, 1000 nm.

The invention is particularly efficient with particles of a diameter of about 500 - 800 nm, preferably about 500 - 700 nm, because of the microscopic structure of the hair cuticle (outer cuticle), which is the outer cortical layer of the hair and consists of overlapping cells that lie above the hair medulla (cortex pilii). The cuticular hair can be compared to a periodic asymmetric ratchet-shaped surface and is has been shown that oscillating radial hair motion induces directed nanoparticle transport into the hair follicle with maximal velocity at a specific optimal frequency and an optimal particle size of about 500 - 700 nm. However, the skilled person understands that the respective anatomical structures responsible for the advantageous effect of using certain particle sizes can depend on the body region of the hair follicle and on the species of the subject.

In the context of the invention, a submicron particle comprised by a composition of the invention has to be stable for the period of applying the composition to the skin of a subject. For example, in embodiments, the particles may dissolve after a certain period. However, during the process of applying the composition of the invention to a skin area containing hair follicles, preferably by massaging the skin in a way that induces radial hair motion, the particles remain solid/stable and maintain a size in the range of 100-1000 nm.

In embodiments, the submicron particle of the invention are sufficiently stable to enable the interaction with the hair cuticle to enable the ratchet effect described in the art. This can be achieved with solid, semi-solid and soft submicron particles, such as soft, semi-solid or solid nanoparticles. In preferred embodiments, the particles are solid or semi-solid.

Accordingly, the particles of the invention may (slowly) dissolve in the dispersion medium and/or in the hair follicle and/or on the skin after application. The skin temperature, which may be higher than the storage temperature of the composition, may induce the dissolving process. However, the composition, dispersion medium and/or particles are selected in a way to ensure sufficient stability of the particles during the initial phase after application of the composition to the skin while the particles and the dispersion medium with the dissolved active agent enter into the hair follicle.

In embodiments, the submicron particles comprised in the composition of the invention do not comprise or consist of an active agent. The particles may be stable and may not dissolve or disintegrate after application to the skin of a subject, or the particles may dissolve or disintegrate after a certain time after application to the skin and/or penetration of the hair of the hair follicle. In embodiments, the particles do not dissolve after administration.

In further embodiments, the submicron particles are nanocrystals or lipid nanoparticles. Nanoparticle drug delivery systems are known in the art and are engineered technologies that use nanoparticles for the targeted delivery and controlled release of therapeutic agents. In such embodiments, the particles of the composition may be used to administer a further active agent to the hair follicle in addition to the active agent dissolved in the dispersion medium.

In specific embodiments, the active agent comprises or is a small molecule and/or a large molecule.

As shown in the examples below, the present invention works equally efficient in delivering small chemical compounds as well as biopharmaceutical active agents, such as protein, antibodies, or nucleic acid molecules to the hair follicle. Accordingly, the present invention is not limited to a specific subclass or kind of active agents or molecules but works for almost all known active agents that can be dissolved in a suitable solvent or solvent composition.

Accordingly, the invention can be used for treating all kinds of conditions for which the skin and in particular the hair follicle represents a suitable route of administration and entry point into the body of the subject to be treated.

Therefore, in one aspect, the invention relates to a pharmaceutical composition of the invention for use in the treatment and/or prophylaxis of hair loss. The invention can be used to directly deliver active agents that influence the function of the hair follicle and the anatomical structures and components comprised by the hair follicle that are involved in maintaining hair and hair growth.

In embodiments, the active agent of the composition directly acts on structures or cells that are comprised by the hair follicle and that are involved in its function.

In a further embodiment, the pharmaceutical composition of the invention can be for use in the treatment and/or prophylaxis of acne.

In further embodiment, the pharmaceutical composition of the invention can be for use in the treatment and/or prophylaxis of folliculitis.

Acne and folliculitis are examples of conditions that involve hair follicles and in which a direct and more efficient delivery of active agents to this specific anatomical structure may be particularly advantageous.

Furthermore, in embodiments the pharmacologically active therapeutic and/or prophylactic agent is a vaccine. In further exemplary embodiments, the pharmacologically active therapeutic and/or prophylactic agent is an analgetic, such as tetracaine or lidocaine, a disinfectant, an antibiotic, such as minocyclin, nicotine, fentanyl, morphin, or a glukocorticoide.

In embodiments, the composition is applied to an area of skin having hair follicles. In embodiments, the composition is suitable for application to an area of skin having hair follicles. In embodiments, the topical dispersion medium is suitable for application to a skin area having hair follicles.

In a further aspect, the composition of the invention is a cosmetic composition comprising a cosmetically active agent. Compositions as disclosed herein may be used for solely cosmetic purposes even in subjects that do not suffer from a medical condition.

In embodiments, the compositions of the invention are used for the purpose of improving or changing the outer appearance of a subject, for example for changing the appearance of the skin or the hair of a subject.

In embodiments, the invention may be used to reduce or remove hair from a skin area. This removal may be for cosmetic or medical purposes.

The invention also relates to a cosmetic method comprising
e. the provision of a cosmetic composition of matter according to claim 12,
f. the application of the cosmetic composition to an area of skin with hair follicles.

Cosmetic applications of the present invention comprise, without limitation, products and applications/methods for acne prone skin, skin cleansing, hair cleansing, hair care, skin tint, skin disinfection, skin care, anti-aging products, anti-redness (products for rosacea-prone skin), whitening products, long-lasting products, make-up, non-permanent tattoos, moisturizers, hyaluronic acid, antioxidants.

All features and embodiments that are disclosed in the context of one aspect of the invention described herein are herewith also disclosed in the context of the other aspects of the invention. For example, if a feature is disclosed in the context of the pharmaceutical composition of the invention, the respective feature can also be present in the context of a cosmetic composition or cosmetic method as disclosed herein, and the other way around.

### DETAILED DESCRIPTION OF THE INVENTION

The present invention is directed to a composition for the transport of a dissolved active agent into hair follicles, comprising an active agent dissolved in a topical dispersion medium, and submicron particles. Preferably, the compositions of the invention are for application to mammalian, preferably human subjects.

As used herein, "transport" relates to delivery of an active agent to the hair follicle. This transport or delivery process relates to the penetration of the dissolved active agent, preferably together with the dissolving dispersion medium, into the hair follicle. Accordingly, in the context of the invention, transport or delivery of an active agent into a hair follicle describes the process of the active agent entering into the hair follicle after application of the composition of the invention to a skin area comprising hair follicles. As described above, in embodiments penetration of the follicle by the particles of the composition of the invention can be enhanced by inducing a radial movement of the hair, for example by massaging the respective skin area after application of the composition. It was surprisingly found that entry and penetration of the follicle by the particles is accompanied by entry of the dissolved active agent although the agent is not bound to the particles.

As used herein, the term "hair follicle" refers to the anatomical structure of the skin comprising a hair. The hair follicle is an organ found in mammalian skin that resides in the dermal layer of the skin and is made up of 20 different cell types, each with distinct functions. The hair follicle regulates hair growth via a complex interaction between hormones, neuropeptides, and immune cells. This complex interaction induces the hair follicle to produce different types of hair as seen on different parts of the body. For example, terminal hairs grow on the scalp and lanugo hairs are seen covering the bodies of fetuses in the uterus and in some newborn babies. The process of hair growth occurs in distinct sequential stages. The first stage is called anagen and is the active growth phase, telogen is the resting stage, catagen is the regression of the hair follicle phase, exogen is the active shedding of hair phase and lastly kenogen is the phase between the empty hair follicle and the growth of new hair.

The function of hair in humans has long been a subject of interest and continues to be an important topic in society, developmental biology and medicine. Of all mammals, humans have the longest growth phase of scalp hair compared to hair growth on other parts of the body. For centuries, humans have ascribed esthetics to scalp hair styling and dressing and it is often used to communicate social or cultural norms in societies. In addition to its role in defining human appearance, scalp hair also provides protection from UV sun rays and is an insulator against extremes of hot and cold temperatures. Differences in the shape of the scalp hair follicle determine the observed ethnic differences in scalp hair appearance, length and texture.

There are many human diseases in which abnormalities in hair appearance, texture or growth are early signs of local disease of the hair follicle or systemic illness. Well known diseases of the hair follicle include alopecia or hair loss, hirsutism or excess hair growth and lupus erythematosus. Such diseases associated with abnormalities in hair appearance, texture or growth may be treated using compositions of the invention.

The position and distribution of hair follicles changes over the body. For example, the skin of the palms and soles does not have hair follicles whereas skin of the scalp, forearms, legs and genitalia has abundant hair follicles.

There are many structures that make up the hair follicle. Anatomically, the triad of hair follicle itself, sebaceous gland and arrector pili muscle make up the pilosebaceous unit. However, as used herein, the term "hair follicle" comprises all three components of pilosebaceous unit and includes the sebaceous gland and the muscle.

A hair follicle comprises the papilla, the hair matrix, a root sheath and the bulge. Other structures associated with the hair follicle and comprised by the term as used herein include the cup or funnel in which the follicle grows known as the infundibulum, the arrector pili muscles, the sebaceous glands, and the apocrine sweat glands. Hair follicle receptors sense the position of the hair.

The papilla is a large structure at the base of the hair follicle. The papilla is made up mainly of connective tissue and a capillary loop. Blood vessels in the dermal papillae nourish all hair follicles and bring nutrients and oxygen to the lower layers of epidermal cells. Around the papilla is the hair matrix.

The root sheath is composed of an external and internal root sheath. The external root sheath appears empty with cuboid cells when stained with H&E stain. The internal root sheath is composed of three layers, Henle's layer, Huxley's layer, and an internal cuticle that is continuous with the outermost layer of the hair fiber.

The bulge is located in the outer/external root sheath at the insertion point of the arrector pili muscle. It houses several types of stem cells, which supply the entire hair follicle with new cells, and take part in healing the epidermis after a wound. Stem cells express the marker LGR5+ in vivo.

Attached to the follicle is a tiny bundle of muscle fiber called the arrector pili. This muscle is responsible for causing the follicle lissis to become more perpendicular to the surface of the skin and causing the follicle to protrude slightly above the surrounding skin (piloerection) and a pore encased with skin oil.

Also attached to the follicle is a sebaceous gland, which produces the oily or waxy substance sebum. The higher the density of the hair, the more sebaceous glands that are found. The sebaceous gland is a microscopic exocrine gland in the skin that opens into a hair follicle to secrete an oily or waxy matter, called sebum, which lubricates the hair and skin of mammals. Sebaceous glands secrete the oily, waxy substance called sebum that is made of triglycerides, wax esters, squalene, and metabolites of fat-producing cells. Sebaceous secretions in conjunction with apocrine glands also play an important thermoregulatory role. In hot conditions, the secretions emulsify the sweat produced by the eccrine glands and this produces a sheet of sweat that is not readily lost in drops of sweat. This is of importance in delaying dehydration. In colder conditions, the nature of sebum becomes more lipid, and in coating the hair and skin, rain is effectively repelled. Sebum is produced in a holocrine process, in which cells within the sebaceous gland rupture and disintegrate as they release the sebum and the cell remnants are secreted together with the sebum. The cells are constantly replaced by mitosis at the base of the duct. Sebum, secreted by the sebaceous gland in humans, is primarily composed of triglycerides (≈41%), wax esters (≈26%), squalene (≈12%), and free fatty acids (≈16%), and cholesterol. The composition of sebum varies across species. Wax esters and squalene are unique to sebum and not produced as final products anywhere else in the body. Sapienic acid is a sebum fatty acid that is unique to humans, and is implicated in the development of acne. Sebum is odorless, but its breakdown by bacteria can produce strong odors. Sex steroids are known to affect the rate of sebum secretion; androgens such as testosterone have been shown to stimulate secretion, and estrogens have been shown to inhibit secretion. Dihydrotestosterone acts as the primary androgen in the prostate and in hair follicles.

In embodiments, compositions of the invention can be used to deliver active agents to the sebaceous glands and to modify or influence the function of the glands and/or the composition of the sebum, and/or to treat conditions associated with the sebaceous gland function. For example,

In humans, sebaceous glands occur in the greatest number on the face and scalp, but also on all parts of the skin except the palms of the hands and soles of the feet. In the eyelids, meibomian glands, also called tarsal glands, are a type of sebaceous gland that secrete a special type of sebum into tears. Several related medical conditions involve sebum-including acne, hyperplasia, and sebaceous adenoma. These are usually attributable to overactive sebaceous glands, which produce excess sebum. Sebaceous glands are involved in skin problems such as acne and keratosis pilaris. In the skin pores, sebum and keratin can create a hyperkeratotic plug called a comedo.

Acne is a very common problem, particularly during puberty in teenagers, and is thought to relate to an increased production of sebum due to hormonal factors. The increased production of sebum can lead to a blockage of the sebaceous gland duct. This can cause a comedo (commonly called a blackhead or a whitehead), which can lead to infection, particularly by the bacteria *Cutibacterium acnes.* This can inflame the comedones, which then change into the characteristic acne lesions. Comedones generally occur on the areas with more sebaceous glands, particularly the face, shoulders, upper chest and back. Comedones may be "black" or "white" depending on whether the entire pilosebaceous unit, or just the sebaceous duct, is blocked. Sebaceous filaments (innocuous build-ups of sebum) are often mistaken for whiteheads. There are many treatments available for acne from reducing sugars in the diet, to medications that include antibiotics, benzoyl peroxide, retinoids, and hormonal treatments. Retinoids reduce the amount of sebum produced by the sebaceous glands. Should the usual treatments fail, the presence of the Demodex mite could be looked for as the possible cause. Current treatment options may be modified in view of the present invention, which allows an improved delivery of dissolve active agents against acne to the hair follicle and/or particularly to the sebaceous gland or its duct.

Further conditions that involve the sebaceous gland and may be treated by compositions of the invention include Seborrhoea, which is caused by overactive sebaceous glands causing oily skin or hair; Sebaceous hyperplasia, referring to excessive proliferation of the cells within the glands, and visible macroscopically as small papules on the skin, particularly on the forehead, nose and cheeks; Seborrhoeic dermatitis, a chronic, usually mild form of dermatitis effected by changes in the sebaceous glands; Seborrheic-like psoriasis (also known as "Sebopsoriasis" and "Seborrhiasis"), which is a skin condition characterized by psoriasis with an overlapping seborrheic dermatitis; Sebaceous adenoma, a benign slow-growing tumour-which may, however, in rare cases be a precursor to a cancer syndrome known as Muir-Torre syndrome; Sebaceous carcinoma, an uncommon and aggressive cutaneous tumour; Sebaceous cyst, which is a term used to refer to both an epidermoid cyst and a pilar cyst, though neither of these contain sebum, only keratin and do not originate in the sebaceous gland and so are not true sebaceous cysts; Nevus sebaceous, a hairless region or plaque on the scalp or skin, caused by an overgrowth of sebaceous glands; Phymatous rosacea, which is a cutaneous condition characterized by an overgrowth of sebaceous glands.

The form, including the size and shape, of hair follicles has been studied in detail in the art (Vogt et al; Experimental Dermatology 2007, 16, 946-950). However, the size and distribution of hair follicles varies in different body sites, as known to the skilled person (Otberg et al. J Invest Dermatol 122:14 -19, 2004).

The invention maybe used to treat or prevent hair loss. Hair loss, also known as alopecia or baldness, refers to a loss of hair from part of the head or body. Typically, at least the head is involved. The severity of hair loss can vary from a small area to the entire body. Inflammation or scarring is not usually present. Hair loss in some people causes psychological distress. Depending on the case, hair loss may be a medical or a cosmetic problem.

Common types of hair loss include male- or female-pattern hair loss, alopecia areata, and a thinning of hair known as telogen effluvium. The cause of male-pattern hair loss is a combination of genetics and male hormones; the cause of female pattern hair loss is unclear; the cause of alopecia areata is autoimmune; and the cause of telogen effluvium is typically a physically or psychologically stressful event. Telogen effluvium is very common following pregnancy. Less common causes of hair loss without inflammation or scarring include the pulling out of hair, certain medications including chemotherapy, HIV/AIDS, hypothyroidism, and malnutrition including iron deficiency. Causes of hair loss that occurs with scarring or inflammation include fungal infection, lupus erythematosus, radiation therapy, and sarcoidosis. Diagnosis of hair loss is partly based on the areas affected.

Treatment of pattern hair loss may simply involve accepting the condition. Interventions that can be tried include the medications minoxidil (or finasteride) and hair transplant surgery. Alopecia areata may be treated by steroid injections in the affected area, but these need to be frequently repeated to be effective. Hair loss is a common problem. Pattern hair loss by age 50 affects about half of men and a quarter of women. About 2% of people develop alopecia areata at some point in time.

Furthermore, the present invention may be used specifically for cosmetic applications that influence the outer appearance hair follicles and its components, in particular the appearance of the hair (for example its color or structure), or that influence the appearance of the skin by modifying the function of the follicle.

Furthermore, the invention can be used in therapeutic or cosmetic applications that are not directly linked to the function of the hair follicle. For example, the hair follicle may be used as a route of entry or as a reservoir for a pharmacologically or cosmetically active agent.

As used herein, it is understood that an "active agent" or an "active ingredient" is a molecule or ingredient of the composition of the invention, which is biologically active. In the context of the invention, the active agent does not necessarily have to be proven to be biologically active, but it may also be though to have a biological activity, or it may have an alleged biolgocial activity. In the context of a pharmaceutical composition, the term active pharmaceutical ingredient (API) may be used. The term also comprises homeopathic ingredients or other molecules or ingredients with an alleged or proven biological activity. Instead of active agent, the terms active pharmaceutical ingredient (also abbreviated as API) and bulk active may be used in medicine, and the term active substance may be used for natural products. Some medication products may contain more than one active agent. The terms active constituent or active principle are often chosen when referring to the active agent of interest in a plant (such as salicylic acid in willow bark or arecoline in areca nuts), because the word ingredient in many minds connotes a sense of human agency (that is, something that a person combines with other substances), whereas the natural products present in plants were not added by any human agency but rather occurred naturally. However, such plant derived agents may serve as active agents in the context of the composition of the present invention. In contrast to active agents, inactive agents or ingredients are usually called excipients in pharmaceutical contexts. The main excipient that serves as a medium for conveying the active agent is usually called the vehicle.

As used herein, the term active agent comprises small molecules as well as large molecules, in particular biopharmaceuticals.

The term "small molecule" relates to a low molecular weight (preferably <900 daltons) compound that are preferably organic molecules and that may help regulate a biological process. Small molecules can for example act as antagonists to surface enzyme-linked receptors and receptors, or even inhibit metabolic enzymes. Such molecules have been shown to directly inhibit the signaling initiated by the respective ligands binding to their receptors, to recruit antibodies and other immunomodulatory molecules, or to promote or inhibit the proliferation of different immune cells to target specific types of cancer cells. A small molecule preferably has a size on the order of around 1 nm. Many drugs are small molecules. Larger structures such as nucleic acids and proteins, and many polysaccharides are not small molecules, although their constituent monomers (ribo- or deoxyribonucleotides, amino acids, and monosaccharides, respectively) are often considered small molecules. In the context of a pharmaceutical composition, the term "small molecule" may relate to molecules that bind specific biological macromolecules and act as an effector, altering the activity or function of the target. Small molecules can have a variety of biological functions or applications, serving as cell signaling molecules, drugs in medicine, pesticides in farming, and in many other roles. These compounds can be natural (such as secondary metabolites) or artificial (such as antiviral drugs); they may have a beneficial effect against a disease (such as drugs) or may be detrimental (such as teratogens and carcinogens).

Large molecules, such as biopharmaceuticals, also known as a biologic(al) medical products, biologicals, or biologics, include any pharmaceutical drug product manufactured in, extracted from, or semisynthesized from biological sources. They include without limitation vaccines, blood, or blood components, and recombinant therapeutic protein such as antibodies, cytokines, chemokines, and fusion proteins. Biopharmaceuticals can be composed of sugars, proteins, or nucleic acids or complex combinations of these substances, such as viral vectors. They are isolated from natural sources, including humans, animals, cell culture systems or microorganisms.

In the context of the present invention, the active agent is "dissolved" in the topical dispersion medium. It is understood that "dissolved" relates to the active agent being in solution in the medium or a component of the medium, for example in case of a medium that comprises several ingredients with different solubility properties.

In chemistry, a solution is a special type of homogeneous mixture composed of two or more substances. In such a mixture, a solute (here the active agent), is a substance dissolved in another substance, known as a solvent (here the topical dispersion medium or at least one component thereof). The mixing process of a solution happens at a scale where the effects of chemical polarity are involved, resulting in interactions that are specific to solvation. The solution usually has the state of the solvent when the solvent is the larger fraction of the mixture, as is commonly the case. One important parameter of a solution is the concentration, which is a measure of the amount of solute in a given amount of solution or solvent. The term "aqueous solution" is used when one of the solvents is water.

Preferably, the active agent and at least one component of the topical dispersion medium (solvent) form a solution that is a homogeneous mixture of two or more substances. In embodiments, the active agent in the solvent cannot be seen by the naked eye and the solution of the active agent in the solvent is stable and cannot be separated by filtration (or mechanically). In embodiments, the active agent is stained or marked, for example by coupling of a fluorescent marker.

In preferred embodiments, the active agent is not chemically coupled to the submicron particles. In the context of the invention, there is no specific interaction between the submicron particles of the invention and the active agent in the dispersion medium. In embodiments, there is no intended interaction between the particles and the active agent in the dispersion medium. In embodiments, there is no specific interaction between the active agents and the particles, such as an interaction that is actively invoked by the developer of the composition. In particular, in embodiments there is no chemical linkage, like a chemical bond, or any functionalization on the surface of the particles that mediates a specific interaction between the components.

The dispersion medium of the invention comprises submicron particles, which are dispersed therein. A dispersion is a system in which distributed particles of one material are dispersed in a continuous phase of another material, here the dispersion medium comprising the dissolved active agent. In general, the two phases of a dispersion may be in the same or different states of matter. Dispersions are classified in a number of different ways, including how large the particles are in relation to the particles of the continuous phase, whether or not precipitation, agglomeration, flocculation, and/or coalescence occurs, and the presence of Brownian motion.

The submicron particles of the invention have a diameter of 1 µm or less and comprise any kind of particle which is sufficiently rigid to be maintained during topical administration to the skin which preferably involves massaging the skin. This includes soft and solid particles, in particular nanoparticles, such as nanocrystals and lipid nanoparticles. The particles of the composition of the invention have to stay stable and rigid for a sufficient time after application to the skin of a subject to penetrate to the hair follicle. In embodiments, the particles are stable for at least 10 minutes after topical application to the skin. In embodiments, the particles are stable for at least 1 minute after topical application to the skin. In embodiments, the particles are stable for at least 1, 2, 3, 4, 5, 6, 7, 8, 9, 10 or more minutes after topical application to the skin.

In embodiments, the submicron particles are nanoparticles. A nanoparticle (or ultrafine particle) is usually defined as a particle of matter that is between 1 and 100 nanometres (nm) in diameter. However, in the context of the invention the term nanoparticle may also relate to particles larger than 100 nm, but not larger than 1000 nm. Being more subject to the brownian motion, they usually do not sediment, like colloidal particles that conversely are usually understood to range from 1 to 1000 nm.

Dispersions of nanoparticles in transparent media can be transparent, whereas suspensions of larger particles usually scatter some or all visible light incident on them. Nanoparticles also easily pass through common filters, such as common ceramic candles, so that separation from liquids requires special nanofiltration techniques. The properties of nanoparticles often differ markedly from those of larger particles of the same substance. Since the typical diameter of an atom is between 0.15 and 0.6 nm, a large fraction of the nanoparticle's material lies within a few atomic diameters from its surface. Therefore, the properties of that surface layer may dominate over those of the bulk material. This effect is particularly strong for nanoparticles dispersed in a medium of different composition since the interactions between the two materials at their interface also becomes significant.

Nanoparticles occur widely in nature and are objects of study in many sciences such as chemistry, physics, geology and biology. Being at the transition between bulk materials and atomic or molecular structures, they often exhibit phenomena that are not observed at either scale. The production of nanoparticles with specific properties is an important branch of nanotechnology.

In general, the small size of nanoparticles leads to a lower concentration of point defects compared to their bulk counterparts, but they do support a variety of dislocations that can be visualized using high-resolution electron microscopes. However, nanoparticles exhibit different dislocation mechanics, which, together with their unique surface structures, results in mechanical properties that are different from the bulk material.

In the context of pharmaceutical and cosmetic applications, nanoparticles have been used as delivery systems for active agents, such as drugs. Such delivery systems are engineered technologies that use nanoparticles for the targeted delivery and controlled release of active agents. The modern form of a drug delivery system should minimize side-effects and reduce both dosage and dosage frequency. Nanomaterials and nanoparticles exhibit different chemical and physical properties, or biological effects compared to larger-scale counterparts that can be beneficial for drug delivery systems. Some important advantages of nanoparticles are their high surface-area-to-volume ratio, chemical and geometric tunability, and their ability to interact with biomolecules to facilitate uptake across the cell membrane. The large surface area also makes it possible to include a large affinity for drugs and small molecules, like ligands or antibodies, for targeting and controlled release purposes.

Nanoparticles refer to a large family of materials both organic and inorganic. Each material has uniquely tunable properties and thus can be selectively designed for specific applications. Finetuning nanoparticle properties for effective drug delivery involves addressing the following factors. The surface-area-to-volume ratio of nanoparticles can be altered to allow for more ligand binding to the surface. Increasing ligand binding efficiency can decrease dosage and minimize nanoparticle toxicity. Minimizing dosage or dosage frequency also lowers the mass of nanoparticle per mass of drug, thus achieving greater efficiency. Surface functionalization of nanoparticles is another important design aspect and is often accomplished by bioconjugation or passive adsorption of molecules onto the nanoparticle surface. By functionalizing nanoparticle surfaces with ligands that enhance drug binding, suppress immune response, or provide targeting/controlled release capabilities, both a greater efficacy and lower toxicity are achieved. Efficacy is increased as more drug is delivered to the target site, and toxic side effects are lowered by minimizing the total level of drug in the body. The composition of the nanoparticle can be chosen according to the target environment or desired effect. For example, liposome-based nanoparticles can be biologically degraded after delivery, thus minimizing the risk of accumulation and toxicity after the therapeutic cargo has been released. Metal nanoparticles, such as gold nanoparticles, have optical qualities (also described in nanomaterials) that allow for less invasive imaging techniques. Furthermore, the photothermal response of nanoparticles to optical stimulation can be directly utilized for tumor therapy.

As used herein, the term nanoparticles includes polymeric nanoparticles, inorganic nanoparticles, organic nanoparticles, and lipid-based nanoparticles, for example. In embodiments, nanoparticles are drug nanocrystals, which consist of an active compound.

Polymeric nanoparticles are synthetic polymers with a size ranging from 10 to 100 nm. Common synthetic polymeric nanoparticles include polyacrylamide, polyacrylate, and chitosan. Drug molecules can be incorporated either during or after polymerization. Depending on the polymerization chemistry, the drug can be covalently bonded, encapsulated in a hydrophobic core, or conjugated electrostatically. Common synthetic strategies for polymeric nanoparticles include microfluidic approaches, electrodropping, high pressure homogenization, and emulsion-based interfacial polymerization. Polymer biodegradability is an important aspect to consider when choosing the appropriate nanoparticle chemistry. Nanocarriers composed of biodegradable polymers undergo hydrolysis in the body, producing biocompatible small molecules such as lactic acid and glycolic acid. Polymeric nanoparticles can be created via self-assembly or other methods such as particle replication in nonwetting templates (PRINT) which allows customization of composition, size, and shape of the nanoparticle using tiny molds.

Dendrimers are unique hyper-branched synthetic polymers with monodispersed size, well-defined structure, and a highly functionalized terminal surface. They are typically composed of synthetic or natural amino acid, nucleic acids, and carbohydrates. Therapeutics can be loaded with relative ease onto the interior of the dendrimers or the terminal surface of the branches via electrostatic interaction, hydrophobic interactions, hydrogen bonds, chemical linkages, or covalent conjugation. Drug-dendrimer conjugation can elongate the half-life of drugs. Currently, dendrimer use in biological systems is limited due to dendrimer toxicity and limitations in their synthesis methods. Dendrimers are also confined within a narrow size range (<15 nm) and current synthesis methods are subject to low yield. The surface groups will reach the de Gennes dense packing limit at high generation level, which seals the interior from the bulk solution - this can be useful for encapsulation of hydrophobic, poorly soluble drug molecules. The seal can be tuned by intramolecular interactions between adjacent surface groups, which can be varied by the condition of the solution, such as pH, polarity, and temperature, a property which can be utilized to tailor encapsulation and controlled release properties.

Inorganic nanoparticles have emerged as highly valuable functional building blocks for drug delivery systems due to their well-defined and highly tunable properties such as size, shape, and surface functionalization. Inorganic nanoparticles have been largely adopted to biological and medical applications ranging from imaging and diagnoses to drug delivery. Inorganic nanoparticles are usually composed of inert metals such as gold and titanium that form nanospheres, however, iron oxide nanoparticles have also become an option.

Quantum dots (QDs), or inorganic semiconductor nanocrystals, have also emerged as valuable tools in the field of bionanotechnology because of their unique size-dependent optical properties and versatile surface chemistry. Their diameters (2 - 10 nm) are on the order of the exciton Bohr radius, resulting in quantum confinement effects analogous to the "particle-in-a-box" model. As a result, optical and electronic properties of quantum dots vary with their size: nanocrystals of larger sizes will emit lower energy light upon fluorescence excitation. Surface engineering of QDs is crucial for creating nanoparticle-biomolecule hybrids capable of participating in biological processes. Manipulation of nanocrystal core composition, size, and structure changes QD photo-physical properties Designing coating materials which encapsulate the QD core in an organic shell make nanocrystals biocompatible, and QDs can be further decorated with biomolecules to enable more specific interaction with biological targets. The design of inorganic nanocrystal core coupled with biologically compatible organic shell and surface ligands can combine useful properties of both materials, i.e. optical properties of the QDs and biological functions of ligands attached.

Organic nanocrystals consist of pure drugs and surface-active agents required for stabilization. They are defined as carrier-free submicron colloidal drug delivery systems with a mean particle size in the nanometer range. The primary importance of the formulation of drugs into nanocrystals is the increase in particle surface area in contact with the dissolution medium, therefore increasing bioavailability. A number of drug products formulated in this way are on the market.

Liposomes are spherical vesicles composed of synthetic or natural phospholipids that self-assemble in aqueous solution in sizes ranging from tens of nanometers to micrometers. The resulting vesicle, which has an aqueous core surrounded by a hydrophobic membrane, can be loaded with a wide variety of hydrophobic or hydrophilic molecules for therapeutic purposes. Liposomes are typically synthesized with naturally occurring phospholipids, mainly phosphatidylcholine. Cholesterol is often included in the formulation to adjust the rigidity of the membrane and to increase stability. Cholesterol-containing liposomes having a high rigidity can function as submicron particles in the sense of the invention. The molecular cargo is loaded through liposome formation in aqueous solution, solvent exchange mechanisms, or pH gradients methods. Various molecules can also be chemically conjugated to the surface of the liposome to alter recognition properties. One typical modification is conjugating polyethyleneglycol (PEG) to the vesicle surface. The hydrophilic polymer prevents recognition by macrophages and decreases clearance. The size, surface charge, and bilayer fluidity also alter liposome delivery kinetics. As the cell membrane itself is composed of phospholipids, liposomes can directly fuse with the membrane and release the cargo into the cytosol, or may enter the cell through phagocytosis or other active transport pathways. Liposomal delivery has various advantages. Liposomes increase the solubility, stability, and uptake of drug molecules. Peptides, polymers, and other molecules can be conjugated to the surface of a liposome for targeted delivery. Conjugating various ligands can facilitate binding to target cells based on the receptor-ligand interaction. Altering vesicle size and surface chemistry can also be tuned to increase circulation time.

Furthermore, the submicron particles of the invention can be (solid) lipid nanoparticles. Solid lipid nanoparticles (SLNs, sLNPs), or lipid nanoparticles (LNPs), are nanoparticles composed of lipids. They are a novel pharmaceutical drug delivery system (and part of nanoparticle drug delivery), and a novel pharmaceutical formulation. A solid lipid nanoparticle is typically spherical with an average diameter between 10 and 1000 nanometers. Solid lipid nanoparticles possess a solid lipid core matrix that can solubilize lipophilic molecules. The lipid core is stabilized by surfactants (emulsifiers). The emulsifier used depends on administration routes and is more limited for parenteral administrations. The term lipid is used here in a broader sense and includes triglycerides (e.g. tristearin), diglycerides (e.g. glycerol bahenate), monoglycerides (e.g. glycerol monostearate), fatty acids (e.g. stearic acid), steroids (e.g. cholesterol), and waxes (e.g. cetyl palmitate). All classes of emulsifiers (with respect to charge and molecular weight) have been used to stabilize the lipid dispersion. It has been found that the combination of emulsifiers might prevent particle agglomeration more efficiently. An SLN is generally spherical in shape and consists of a solid lipid core stabilized by a surfactant. The core lipids can be fatty acids, acylglycerols, waxes, and mixtures of these surfactants. Biological membrane lipids such as phospholipids, sphingomyelins, bile salts (sodium taurocholate), and sterols (cholesterol) are utilized as stabilizers. Biological lipids having minimum carrier cytotoxicity and the solid state of the lipid permit better controlled drug release due to increased mass transfer resistance. Shah et al in their book Lipid Nanoparticles: Production, Characterization and Stability discuss these in detail.

The composition of the invention comprises a topical dispersion medium in which the active agent is dissolved, and the submicron particles are dispersed. The term "dispersion medium" refers to a medium that is suitable to dissolve the active agent of the composition and to disperse the submicron particles. In embodiments, the composition of the invention forms a dispersion comprising the active agent dissolved in the dispersion medium and the submicron particles dispersed in the dispersion medium.

The compositions of the invention are for topical administration. Accordingly, the dispersion medium comprising the dissolved active agent and the dispersed submicron particles must be suitable for topical administration and is therefore referred to as a "topical dispersion medium". The term "topical" relates to a form of administration directly to a body surface. A topical medication is a medication that is applied to a particular place on or in the body. As used herein, topical administration means application to body surfaces, in particular the skin. In preferred embodiments, the composition is for topical administration to the skin in an area comprising hair follicles. Media and composition for topical administration in the sense of the present invention include all kinds of suitable media known to the skilled person, including creams, foams, gels, lotions, and ointments.

As used herein, topical is defined as applied to a localized area of the body surface regardless of the location of the effect of the active agent. By this definition, topical administration also includes transdermal application, where the substance is administered onto the skin to be delivered to the hair follicle, and the active agent may be absorbed into the body to attain systemic distribution.

The manufacturer of a topical product, such as cosmetic or pharmaceutical compositions for topical administration, has total control over the content of the base formulation, which is the dispersion medium. Although containing the same active ingredients, one manufacturer's cream might be more acidic than the next, which could cause skin irritation or change its absorption rate. For example, a vaginal formulation of miconazole antifungal cream might irritate the skin less than an athlete foot formulation of miconazole cream. These variations can, on occasion, result in different outcomes, even though the active ingredient is the same. For example, in case of topical steroid products, no comparative potency labeling exists to ensure equal efficacy between brands of topical steroids (percentage of oil vs water dramatically affect the potency of topical steroid). Studies have confirmed that the potency of some topical steroid products may differ according to manufacturer or brand. A skilled person knows or can identify a suitable dispersion medium for a given combination of an active agent and certain submicron particles.

There are different classes of media for topical administration that can serve as topical dispersion medium in the context of the invention and a skilled person can select a suitable medium depending on the intended use, the active agent and the dispersed submicron particles. There are many general classes of topical media, with no clear dividing line among similar formulations. As a result, what the manufacturer's marketing department chooses to list on the label of a topical product might be completely different from what the form would normally be called.

As used herein, the term "topical dispersion media" comprises, without limitation, a liquid, an oil, a cream, a lotion, a foam, a gel, an ointment, or a mixture of one or more of these, preferably a liquid such as water, ethanol or a surfactant solution.

A liquid is a nearly incompressible fluid that conforms to the shape of its container but retains a (nearly) constant volume independent of pressure. As such, it is one of the four fundamental states of matter (the others being solid, gas, and plasma), and is the only state with a definite volume but no fixed shape. A liquid is made up of tiny vibrating particles of matter, such as atoms, held together by intermolecular bonds. A liquid is able to flow and take the shape of a container. Most liquids resist compression, although others can be compressed. A distinctive property of the liquid state is surface tension, leading to wetting phenomena. Water is, by far, the most common liquid on Earth. Liquids have a variety of uses, as lubricants, solvents. Many liquids are used as solvents, to dissolve other liquids or solids. Solutions are found in a wide variety of applications, for example as solvents and dispersion media for pharmaceutical and cosmetic compositions.

Surfactants are compounds that lower the surface tension (or interfacial tension) between two liquids, between a gas and a liquid, or between a liquid and a solid. Surfactants may act as detergents, wetting agents, emulsifiers, foaming agents, or dispersants. As used herein, a dispersion medium of the invention may be formed or may comprise a surfactant solution. Surfactants are usually organic compounds that are amphiphilic, meaning they contain both hydrophobic groups (their tails) and hydrophilic groups (their heads). Therefore, a surfactant contains both a water-insoluble (or oil-soluble) component and a water-soluble component. Surfactants will diffuse in water and adsorb at interfaces between air and water or at the interface between oil and water, in the case where water is mixed with oil. The water-insoluble hydrophobic group may extend out of the bulk water phase, into the air or into the oil phase, while the water-soluble head group remains in the water phase.

An oil is any nonpolar chemical substance that is a viscous liquid at ambient temperatures and is both hydrophobic (does not mix with water, literally "water fearing") and lipophilic (mixes with other oils, literally "fat loving"). Oils have a high carbon and hydrogen content and are usually flammable and surface active. Most oils are unsaturated lipids that are liquid at room temperature. The general definition of oil includes classes of chemical compounds that may be otherwise unrelated in structure, properties, and uses. Oils may be animal, vegetable, or petrochemical in origin, and may be volatile or non-volatile. They are often used for medical or cosmetic purposes in topical administration media.

A cream is an emulsion of oil and water in approximately equal proportions. It penetrates the stratum corneum outer layer of skin wall. Cream is thicker than lotion, and maintains its shape when removed from its container. It tends to be moderate in moisturizing tendency. For topical steroid products, oil-in-water emulsions are common. Creams have a significant risk of causing immunological sensitization due to preservatives and have a high rate of acceptance by patients. There is a great variation in ingredients, composition, pH, and tolerance among generic brands.

Foam is formed by trapping pockets of gas in a liquid and in topical pharmaceutical compositions can be seen for example with topical steroid marketed for the scalp.

Gels are thicker than other liquids and are often a semisolid emulsion and sometimes use alcohol as a solvent for the active ingredient; some gels liquefy at body temperature. As used herein, gels are comprised by the term liquid. In general, a gel is a semi-solid that can have properties ranging from soft and weak to hard and tough. Gels are defined as a substantially dilute cross-linked system, which exhibits no flow when in the steady-state. A gel has been defined phenomenologically as a soft, solid or solid-like material consisting of two or more components, one of which is a liquid, present in substantial quantity.

Gels for topical administration tend to be cellulose cut with alcohol or acetone. Gels tend to be self-drying, tend to have greatly variable ingredients between brands, and carry a significant risk of inducing hypersensitivity due to fragrances and preservatives. Gel is useful for hairy areas and body folds. In applying gel one should avoid fissures in the skin, due to the stinging effect of the alcohol base. Gel enjoys a high rate of acceptance due to its cosmetic elegance.

A lotion is a low-viscosity topical preparation intended for application to the skin. By contrast, creams and gels have usually higher viscosity, typically due to lower water content. However, in embodiments, water-based gels, such polyacrylate gels, with a high water content can be employed. Lotions are applied to external skin with bare hands, a brush, a clean cloth, or cotton wool. While a lotion may be used as a medicine delivery system, many lotions, especially hand lotions and body lotions and lotion for allergies are meant instead to simply smooth, moisturize, soften and, sometimes, perfume the skin. Some skincare products and cosmetics, such as sunscreen and moisturizer, may be available in multiple formats, such as lotions, gels, creams, or sprays. Lotions are usually oil mixed with water. Lotions can be drying if they contain a high amount of alcohol.

An ointment is a homogeneous, viscous, semi-solid preparation, most commonly a greasy, thick oil (oil 80% - water 20%) with a high viscosity, that is intended for external application to the skin or mucous membranes. Ointments have a water number that defines the maximum amount of water that they can contain. They are used as emollients or for the application of active ingredients to the skin for protective, therapeutic, or prophylactic purposes and where a degree of occlusion is desired. Ointments are used topically on a variety of body surfaces. These include the skin and the mucous membranes of the eye (an eye ointment), chest, vulva, anus, and nose. An ointment may or may not be medicated. Ointments are usually very moisturizing, and good for dry skin. They have a low risk of sensitization due to having few ingredients beyond the base oil or fat, and low irritation risk. There is typically little variability between brands of drugs. They are often disliked by patients due to greasiness. The vehicle of an ointment is known as the ointment base. The choice of a base depends upon the clinical or cosmetic indication for the ointment. The different types of ointment bases are:
Further media for topical administration that can be used as topical dispersion medium in the context of the invention include tinctures and topical solutions. Tinctures are skin preparations that has a high percentage of alcohol that would normally be used as a drug vehicle if drying of the area is desired. Topical solutions can be marketed as drops, rinses, or sprays, are generally of low viscosity, and often use alcohol or water in the base.

In pharmaceutical compositions of the invention, the term "active ingredient" or "API" herein refers to a pharmaceutically active molecule as well as its pharmaceutically acceptable and therapeutically active salts, esters, amides, prodrugs, metabolites, enantiomers, polymorphs, analogs, etc. that induce a desired pharmacological or physiological effect. Terms like "active", "active agent", "active substance" may be used synonymously for "active ingredient".

The composition of the invention preferably comprises an effective amount of the active agent. The terms "effective amount" or "therapeutically effective amount" are used interchangeably, and is defined to mean the amount or quantity of the active agent which is sufficient to elicit an appreciable biological response when administered to the subject. It will be appreciated that the precise dose will depend on the age and condition of the patient, the active agent, the nature of the condition to be treated.

The composition of the invention may comprise, preferably in the topical dispersion medium, one or more excipients. The term "excipient" means a biologically or pharmacologically inactive component such as a diluent, disintegrant, carrier, and the like, of a pharmaceutical or cosmetic product. The excipients that are useful in preparing a cosmetic or pharmaceutical composition are generally safe, and non-toxic.

In the context of a pharmaceutical or cosmetic composition, the composition and/or the topical dispersion medium may comprise an acceptable carrier. The term "acceptable carrier" as used herein refers to a non-toxic the material that is compatible with skin and skin appendages and may be used synonymously with the term topical dispersion medium.

For example, "acceptable carrier" means a carrier that is compatible with skin and acceptable for application to the skin of the body. The acceptable carrier may include, for example, water and/or water-soluble solvents. The carrier is preferably suitable as a medium comprising the active agent and the submicron particles and serving as a kind of buffer solution or medium or material. In embodiments, the carrier is suitable for retaining functionality of active agent. In embodiments, the cosmetically acceptable carrier comprises or is a physiological buffer or medium and may comprise, for example, water and/or water-soluble solvents. Furthermore, the carrier may comprise one or more of glycerin, C1-4 alcohols, organic solvents, fatty alcohols, fatty ethers, fatty esters, polyols, glycols, vegetable oils, mineral oils, liposomes, laminar lipid materials, water, or any combinations thereof. As examples of organic solvents, non-limiting mentions can be made of monoalcohols and polyols such as ethyl alcohol, isopropyl alcohol, propyl alcohol, benzyl alcohol, and phenylethyl alcohol, or glycols or glycol ethers such as, for example, monomethyl, monoethyl and monobutyl ethers of ethylene glycol, propylene glycol or ethers thereof such as, for example, monomethyl ether of propylene glycol, butylene glycol, hexylene glycol, dipropylene glycol as well as alkyl ethers of diethylene glycol, for example monoethyl ether or monobutyl ether of diethylene glycol. Other suitable examples of organic solvents are ethylene glycol, propylene glycol, butylene glycol, hexylene glycol, propane diol, and glycerin. The organic solvents can be volatile or non-volatile compounds.

Furthermore, in embodiments the carrier can comprise a liposomal system, such as liposomes, transferosomes that besides phospholipids, contain a single chain surfactant as an edge activator, ethosomes that contains ethanol as an edge activator, preferably niosomes which contain bile acids called bilosomes. In such embodiments, the active agent of the composition can be formulated (packaged/encapsulated) in the liposomal system. For example, the active agent can be formulated in niosomes to achieve better bioavailability, stability, increased resistance to proteolytic enzymes and/or sustained release to prolong the duration of action. Furthermore, the cosmetic composition of the invention can comprise invasomes for delivering the active agent to the skin. As described herein, the term "invasomes" relates to a topical and transdermal delivery system for hydrophilic and lipophilic compounds. Invasomes are composed of soy-phosphatidylcholine (SPC), lyso-phosphatidylcholine, terpenes and ethanol. SPC forms the invasomal bi-layer matrix and lyso-phosphatidylcholine acts as an edge activator imparting flexibility to the phosphatidylcholine bilayers. Both ethanol and terpenes act as penetration enhancers for bioactive compounds and also impart a fluidity or flexibility to the phospholipid bilayers (Mukul Ashtikar, Transdermal delivery from liposomal formulations - Evolution of the technology over the last three decades, Journal of Controlled Release 242, 126-140, 2016).

In embodiments, the invention relates to a cosmetic compositions and method for improving the appearance of the skin and its appendages. In embodiments, improvement of the appearance of the skin relates to the skin adopting an appearance that makes the subject look younger. For example, wrinkling of the skin that appeared with age may be reversed. Also, local changes of the skin color and the appearance of spots. Intrinsic skin aging is a process of chronologically physiological change. Aging of photo protected areas is mainly due to intrinsic genetic or metabolic factors, whereas exposed skin areas are additionally influenced by extrinsic factors, especially solar UV radiation. The process of skin aging and possibilities of slowing and reversing it have been reviewed by Shoubing Zhang et al (Cell Transplantation 2018, Vol. 27(5) 729-738).

Herein, when using the word "skin", this includes also skin appendages. The cosmetic composition of the invention can reverse certain aspects of skin aging and is therefore suitable for improving the out appearance of the skin. This includes, without limitation, skin adopting an appearance that makes the subject look younger, improving skin elasticity, improving hair growth, preventing and/or reversing the development of skin wrinkles, increasing the elasticity of skin scars and/or remodeling skin scar tissue to adopt the appearance of uninjured skin, for example.

As used herein, the term "subject" includes both human and veterinary subjects. The term "treatment" refers to a therapeutic intervention that ameliorates a sign or symptom of a disease or pathological condition after it has begun to develop. As used herein, the term "ameliorating," with reference to a disease or pathological condition, refers to any observable beneficial effect of the treatment. The beneficial effect can be evidenced, for example, by a delayed onset of clinical symptoms of the disease in a susceptible subject, a reduction in severity of some or all clinical symptoms of the disease, a slower progression of the disease, an improvement in the overall health or well-being of the subject, or by other parameters well known in the art that are specific to the particular disease.

The present invention encompasses both treatment and prophylactic treatment of a subject. A "prophylactic" treatment is a treatment administered to a subject who does not exhibit signs of a disease or exhibits only early signs for the purpose of decreasing the risk of developing pathology.

In embodiments, the invention relates to cosmetic or pharmaceutical compositions prepared for topical administration to a subject and which include an effective amount of one or more of the active agents.

Cosmetic or pharmaceutical compositions of the invention for administration to a subject can include at least one further pharmaceutically acceptable additive such as carriers, thickeners, diluents, buffers, preservatives, surface active agents and the like in addition to the active agent. Pharmaceutical compositions can also include one or more additional active ingredients such as antimicrobial agents, anti-inflammatory agents, anesthetics, and the like. The pharmaceutically or cosmetically acceptable carriers for topical administration (i.e. the topical dispersion medium) useful for these formulations may be conventional and can be selected depending on the specific combination of active agent and submicron particle. Remington's Pharmaceutical Sciences, by E. W. Martin, Mack Publishing Co., Easton, PA, 19th Edition (1995), describes compositions and formulations suitable for pharmaceutical delivery of the compounds herein disclosed.

The compositions of the disclosure can alternatively contain as acceptable carrier substances as required to approximate physiological conditions, such as pH adjusting and buffering agents, tonicity adjusting agents, wetting agents and the like, for example, sodium acetate, sodium lactate, sodium chloride, potassium chloride, calcium chloride, sorbitan monolaurate, and triethanolamine oleate.

"Administration of" and "administering" a composition of the invention should be understood to mean providing the composition to a subject. The composition can be administered by another person to the subject or it can be self-administered by the subject. Dosage can be varied depending on the purpose and active agent, for example by the attending clinician to maintain a desired concentration at a target site. Higher or lower concentrations can be selected based on suitable parameters. Dosage can also be adjusted based on the release rate of the administered formulation.

The present invention also relates to a method of treatment of subjects comprising the application of a composition of the invention to an area of skin with hair follicles. The method of treatment comprises preferably the administration of a therapeutically effective amount of an active agent to a subject in need thereof.

An "effective amount" refers to a quantity of a specified agent sufficient to achieve a desired effect in a subject being treated with that agent comprised in a composition of the invention. The effective amount of an agent will be dependent on the subject being treated, the severity of the affliction, and the manner of administration of the therapeutic composition. Dosage regimens can be adjusted to provide an optimum prophylactic or therapeutic response. A therapeutically effective amount is also one in which any toxic or detrimental side effects of the compound and/or other biologically active agent is outweighed in clinical terms by therapeutically beneficial effects.

The instant disclosure also includes kits, packages and multi-container units containing the herein described cosmetic or pharmaceutical compositions, active ingredients, and/or means for administering the same for use in the prevention and treatment of diseases and other conditions in mammalian, preferably human subjects.

### FIGURES

The invention is further described by the following figures. These are not intended to limit the scope of the invention but represent preferred embodiments of aspects of the invention provided for greater illustration of the invention described herein.

### Brief description of the figures:

**Figure 1****:** Confocal microscopic image of a hair follicle after application of dissolved fluorescein natrium salt in a particle containing dispersion medium.
**Figure 2****:** Results of example 2.
**Figure 3****:** Results of example 3.
**Figure 4****:** Results of example 4.
**Figure 5****:** Results of example 5.
**Figure 6****:** Summary of examples 2-5.

### Detailed description of the figures:

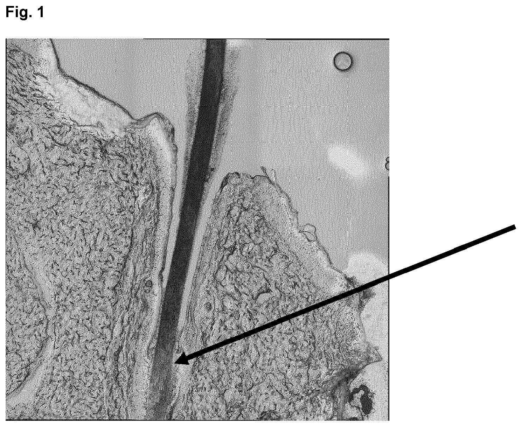

**Figure 1****:** Confocal microscopic image of a skin section with hair follicle after topical application of fluorescein natrium salt (green fluorescent dye) dissolved in dispersion medium together with a caffeine nanosuspension. The localization of the dye can be seen very deep in the hair follicle, as indicated by the arrow.

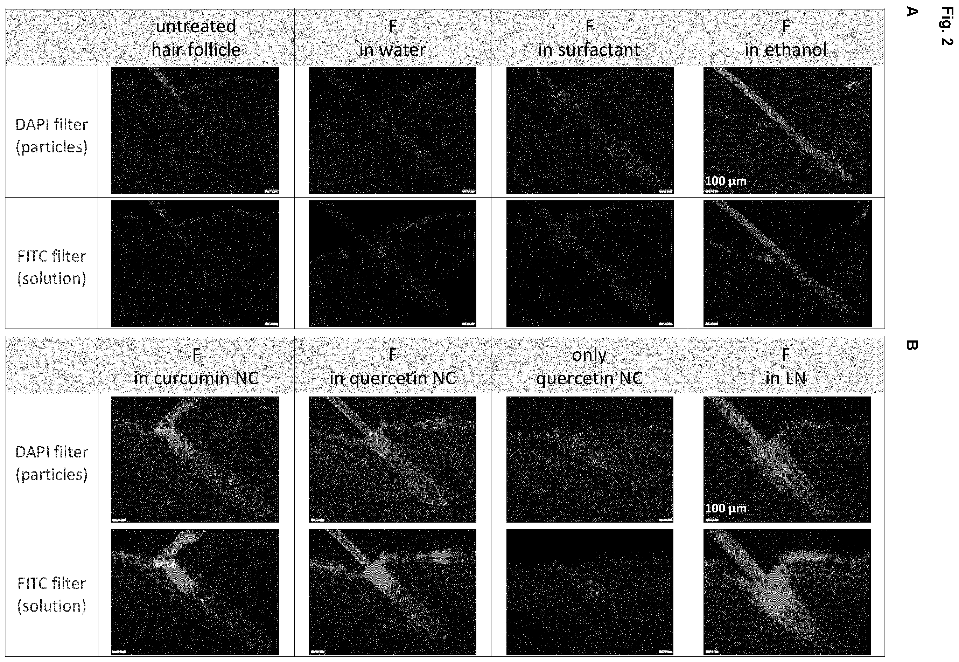

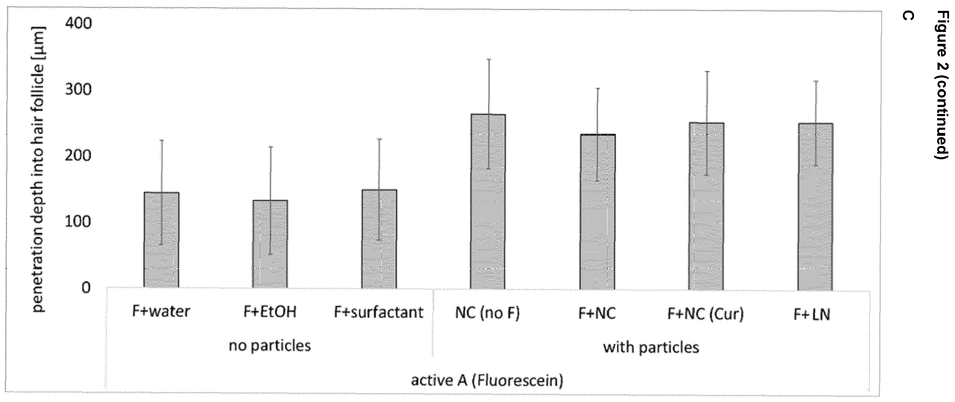

**Figure 2****:** Panels **A** and **B** show exemplary microscopic images using epifluorescence microscopy illustrating the effect of submicron particles on follicle penetration by dissolved fluorescein. **C** The differences in penetration depths between the particle-free and particle-containing formulations are always significant (p< 0.001; *one-way ANOVA with Dunnett post-hoc analysis).* The penetration depth for F with particles is > 160% compared to the particle-free formulations.

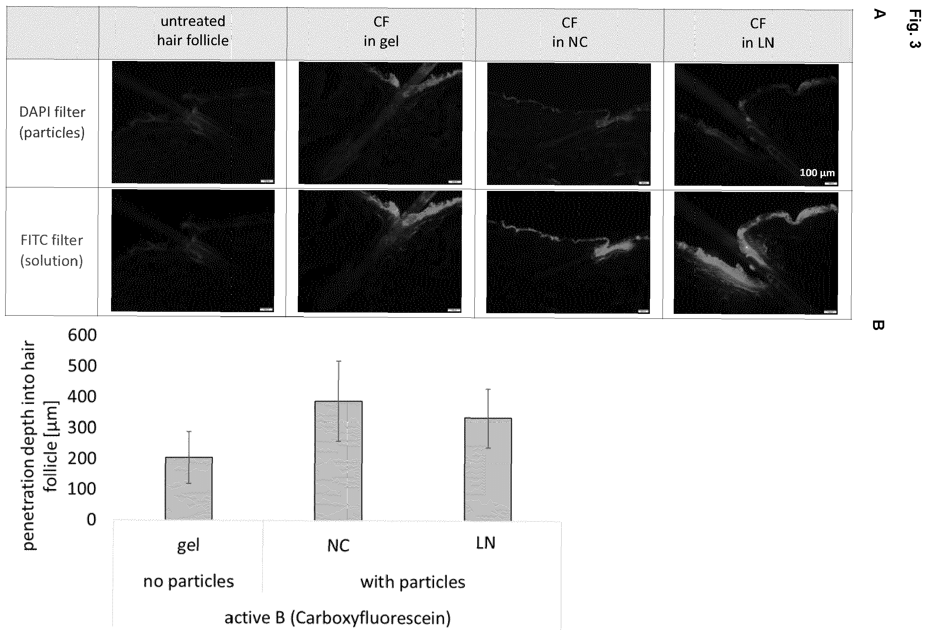

**Figure 3****:** Panel **A** shows exemplary microscopic images using epifluorescence microscopy illustrating the effect of submicron particles on follicle penetration by dissolved carboxyfluoresein. **B** The differences in penetration depths between the particle-free and particle-containing formulations is significant (p< 0.05; *one-way ANOVA with Dunnett post-hoc analysis*). The penetration depth for CF with particles is > 175% compared to the particle-free formulations.

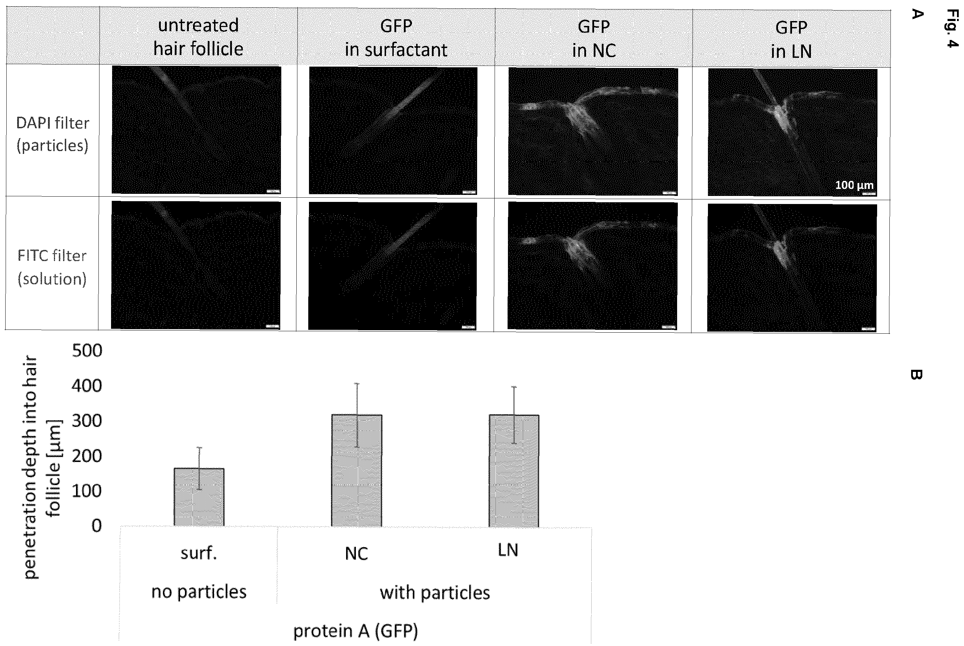

**Figure 4****:** Panel **A** shows exemplary microscopic images using epifluorescence microscopy illustrating the effect of submicron particles on follicle penetration by GFP. **B** The differences in penetration depths between the particle-free and particle-containing formulations are always significant (p< 0.05; *one-way ANOVA with Dunnett post-hoc analysis*). The penetration depth for GFP with particles is > 190% compared to the particle-free formulations.

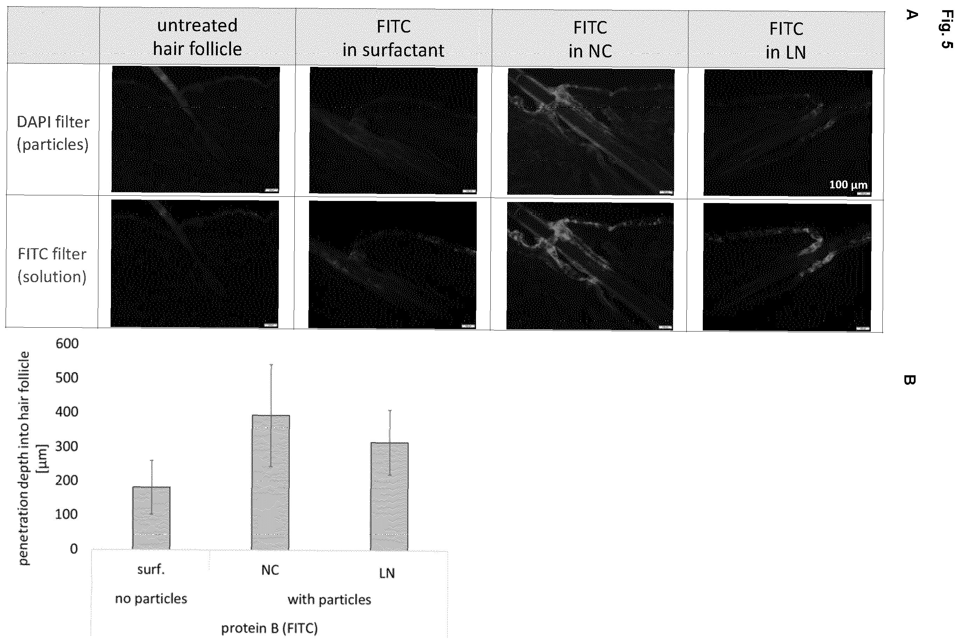

**Figure 5****:** Panel **A** shows exemplary microscopic images using epifluorescence microscopy illustrating the effect of submicron particles on follicle penetration by dissolved FITC-BSA. B The differences in penetration depths between the particle-free and particle-containing formulations are always significant (p< 0.05; *one-way ANOVA with Dunnett post-hoc analysis*). The penetration depth for FITC-BSA with particles is > 190% compared to the particle-free formulations.

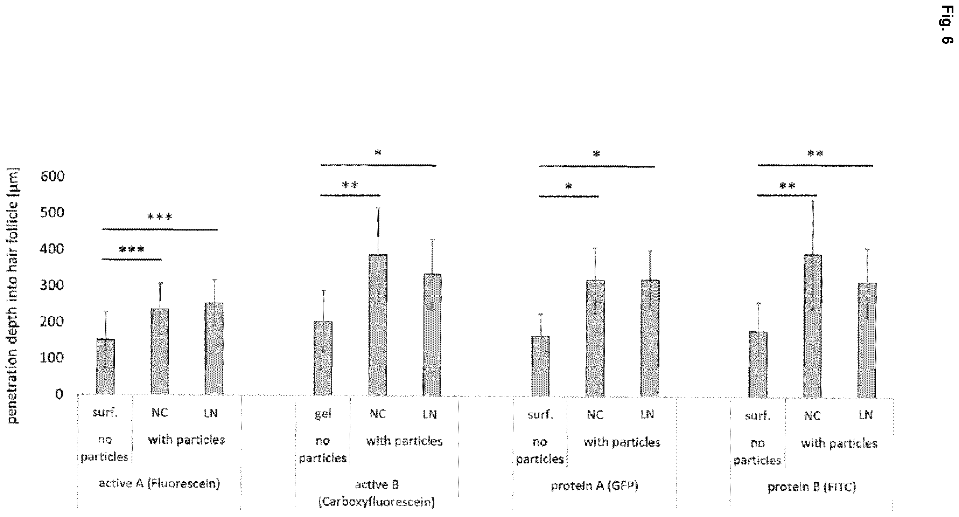

**Figure 6****:** The results of the examples 2-5 are summarized in this figure showing the penetration depth of the different small and large molecules into the hair follicle in µm in the absence and presence of different kinds of submicron particles.

### EXAMPLES

The invention is further described by the following examples. These are not intended to limit the scope of the invention but represent preferred embodiments of aspects of the invention provided for greater illustration of the invention described herein.

### Example 1

Particle-containing formulations were applied as dispersions (particles in a liquid or semi-solid dispersion medium). Besides the particles, the dispersion medium also contained dissolved molecules, name the fluorescent molecule fluorescein, which is an organic compound soluble in water and alcohol. It has been observed that dissolved fluorescein present in the dispersion medium was also transported into the hair follicles when particle-containing formulations were applied. However, if the same medium containing fluorescein was applied without the particles, i.e. only dissolved in the dispersion medium, no efficient penetration into the hair follicles takes place (see Figure 1).

### Example 2 : Particle-mediated delivery of fluorescein (F) as a small molecule drug surrogate (small molecule 1).

Three particle-free and three particle-containing formulations were prepared, each containing 0.003% F (dissolved in dispersion medium), and the penetration of F into hair follicles (HF) was studied. Results are shown in Figure 2.

Particle-free formulations:
- F in water
- F in EtOH
- F in surfactant solution (TPGS 1%)

Particle-containing formulations:
- F + quercetin-nanocrystals (NC); particle concentration of nanocrystals 5% - stabilized with 1% D-α-Tocopherol-Polyethylenglycolsuccinat (TPGS)
- F + curcumin-nanocrystals (NC); particle concentration of nanocrystals 5% - stabilized with 1% TPGS
- F + lipid nanoparticles (LN); particle concentration of LN 10% - stabilized with 1% TPGS

Fluorescein (0.003% w/w) was added to dispersion media that contained no particles (water, TPGS-solution (1% w/w) or ethanol (96% v/v)) and was added to dispersion media that contained particles (1. curcumin nanocrystals (5% w/w dispersed in TPGS solution (1% w/w) - particle size was 257 nm, polydispersity index 0.26 determined by photon correlation spectroscopy); 2. quercetin nanocrystals (5% w/w dispersed in TPGS solution (1% w/w) - particle size was 580 nm, polydispersity index 0.36, determined by photon correlation spectroscopy); 3. Lipid nanoparticles (10% w/w dispersed in TPGS solution (1% w/w) - particle size was 195 nm, polydispersity index 0.09, determined by photon correlation spectroscopy).

The formulations (50 µl on a skin areal of 4 cm²) were applied on fresh porcine ears (ventral side, massage for 60s, penetration time 1h at 32°C). Skin biopsies (Ø 15 mm) were obtained from the treated skin areas from which skin sections (40 µm, 100 sections/biopsy) were prepared. The skin sections and the hair follicle sections were analyzed by epifluorescence microscopy. Images were taken and the penetration depth of the fluorescein was directly measured (n=3).

### Example 3: Particle-mediated delivery of carboxyfluoresein (CF) as a small molecule drug surrogate (small molecule 2).

One particle-free and two particle-containing formulations were prepared, each containing 0.05% CF (dissolved in dispersion medium), and the penetration of CF into HF was investigated. Results are shown in Figure 3.

Particle-free formulations:
- CF in mixture 30% ethanol and 70% propylene glycol with 2% polyacrylic acid Particle-containing formulations:
- CF + quercetin - nanocrystals (NC); particle concentration of nanocrystals 5% - stabilized with 1% TPGS
- CF + lipid nanoparticles (LN); particle concentration of LN 10% - stabilized with 1% TPGS

Carboxyfluorescein (0.05% w/w) was added to a gel that contained a mixture of 30% ethanol and 70% propylene glycol with 2% w/w polyacrylate, but no particles. Carboxyfluorescein (0.05% w/w) was also added to the particle containing formulations (1. quercetin nanocrystals (5% w/w dispersed in TPGS solution (1% w/w) - particle size was 580 nm, polydispersity index 0.36, determined by photon correlation spectroscopy); 3. Lipid nanoparticles (10% w/w dispersed in TPGS solution (1% w/w) - particle size was 195 nm, polydispersity index 0.09, determined by photon correlation spectroscopy).

The formulations (40 µl on a skin areal of 4 cm²) were applied on fresh porcine ears (ventral side, massage for 60s, penetration time 15 min at 32°C). Skin biopsies (Ø 15 mm) were obtained from the treated skin areas from which skin sections (40 µm, 100 sections/biopsy) were prepared. The skin sections and the hair follicle sections were analyzed by epifluorescence microscopy. Images were taken and the penetration depth of the carboxyfluorescein was directly measured (n=3).

### Example 4: Particle-mediated delivery of green fluorescent protein (GFP) as a large molecule drug surrogate (large molecule 3).

One particle-free and two particle-containing formulations were prepared, each containing 0.08% GFP (dissolved in dispersion medium), and the penetration of GFP into HF was investigated. Results are shown in Figure 4.

Particle-free formulations:
- GFP in surfactant solution (TPGS 1%)

Particle-containing formulations:
- GFP + quercetin - nanocrystals (NC); particle concentration of nanocrystals 5% - stabilized with 1% TPGS
- GFP + lipid nanoparticles (LN); particle concentration of LN 10% - stabilized with 1% TPGS

Green fluorescent protein (GFP) (0.08% w/w) was added to a surfactant solution (1% w/w TPGS) without particles. GFP (0.08% w/w) was also added to two particle containing formulations (1. quercetin nanocrystals (5% w/w dispersed in TPGS solution (1% w/w) - particle size was 580 nm, polydispersity index 0.36, determined by photon correlation spectroscopy); 2. Lipid nanoparticles (10% w/w dispersed in TPGS solution (1% w/w) - particle size was 195 nm, polydispersity index 0.09, determined by photon correlation spectroscopy).

The formulations (25 µl on a skin areal of 1,8 cm²) were applied on fresh porcine ears (ventral side, massage for 180s, penetration time 6h at 32°C). Skin biopsies (Ø 15 mm) were obtained from the treated skin areas from which skin sections (40 µm, 100 sections/biopsy) were obtained. The skin sections and the hair follicle sections were analyzed by epifluorescence microscopy. Images were taken and the penetration depth of GFP was directly measured (n=3).

### Example 5: Particle-mediated delivery of FITC-BSA (FITC chemically bound to human serum albumin) as a large molecule drug surrogate (large molecule 4).

One particle-free and two particle-containing formulations were prepared, each with 0.8% FITC-BSA (dissolved in the dispersion medium), and the penetration of FITC-BSA into the HF was investigated. Results are shown in Figure 5.

Particle-free formulations:
- FITC-BSA in surfactant solution (TPGS 1%)

Particle-containing formulations:
- FITC-BSA + quercetin - nanocrystals (NC); particle concentration of nanocrystals 5% - stabilized with 1% TPGS
- FITC-BSA + lipid nanoparticles (LN); particle concentration of LN 10% - stabilized with 1% TPGS

FITC (0.8% w/w) was added to a surfactant solution (1% w/w TPGS) without particles. FITC (0.8% w/w) was also added to two particle containing formulations (1. quercetin nanocrystals (5% w/w dispersed in TPGS solution (1% w/w) - particle size was 580 nm, polydispersity index 0.36, determined by photon correlation spectroscopy); 2. Lipid nanoparticles (10% w/w dispersed in TPGS solution (1% w/w) - particle size was 195 nm, polydispersity index 0.09, determined by photon correlation spectroscopy).

The formulations (25 µl on a skin areal of 1,8 cm²) were applied on fresh porcine ears (ventral side, massage for 180s, penetration time 6h at 32°C). Skin biopsies (Ø 15 mm) were obtained from the treated skin areas from which skin sections (40 µm, 100 sections/biopsy) were obtained. The skin sections and the hair follicle sections were analyzed by epifluorescence microscopy. Images were taken and the penetration depth of FITC was directly measured (n=3).

### Summary of the Examples

The results of the examples 2-5 are summarized in Figure 6.

The data show that the addition of particles can significantly improve the penetration depth of dissolved active ingredients (at least by a factor 2).

The improved penetration with the help of particles occurs both for small chemical molecules (small molecules - examples 1-3) and for protein-based active ingredients (large molecules - examples 4+5).

Both spherical particles (LN) and non-spherical particles (NC) increase the penetration of dissolved active ingredients in the dispersion medium.

### References

1. Skin Pharmacol Physiol. 2006;19(4):232-6. doi: 10.1159/000093119.
2. Eur J Pharm Biopharm. 2011;77(3):465-8. doi: 10.1016/j.ejpb.2010.10.015.
3. J Control Release. 2011;150(1):45-8. doi: 10.1016/j.jconrel.2010.11.015.
4. Eur J Pharm Biopharm. 2017;116(1):125-130. doi.org/10.1016/j.ejpb.2016.10.005

## Claims

1. A composition for the transport of a dissolved active agent into hair follicles, comprising
a. an active agent dissolved in a topical dispersion medium, and
b. submicron particles.

2. A pharmaceutical composition for use in transporting a dissolved pharmacologically active therapeutical and/or prophylactic agent into hair follicles comprising
a. a pharmacologically active therapeutic and/or prophylactic agent dissolved in a topical dispersion medium, and
b. submicron particles.

3. A composition according to any one of the preceding claims, wherein the active agent is not chemically coupled to the submicron particles.

4. A composition according to any one of the preceding claims, wherein the topical dispersion medium is a liquid, an oil, a cream, a lotion, a gel or a mixture of one or more of these, preferably a liquid such as water, ethanol or a surfactant solution.

5. A composition according to any one of the preceding claims, wherein the submicron particles have a diameter of 100-1000 nm, preferably 500-800 nm, most preferably about 600 nm.

6. A composition according to any one of the preceding claims, wherein the submicron particles are nanocrystals or lipid nanoparticles.

7. A composition according to any one of the preceding claims, wherein the active agent comprises a small molecule and/or a large molecule.

8. A pharmaceutical composition according to any one of claims 2-7 for use in the treatment and/or prophylaxis of hair loss.

9. A pharmaceutical composition of any one of claims 2-7 for use in the treatment and/or prophylaxis of acne.

10. A pharmaceutical composition of any one of claims 2-7, wherein the pharmacologically active therapeutic and/or prophylactic agent is a vaccine.

11. A pharmaceutical composition of any one of claims 2-10, wherein the composition is applied to an area of skin having hair follicles.

12. A composition of matter according to claim 1, or 3-7, wherein the composition is a cosmetic composition comprising a cosmetically active agent.

13. Cosmetic method comprising
a. the provision of a cosmetic composition of matter according to claim 12,
b. the application of the cosmetic composition to an area of skin with hair follicles.
